# EUROPEAN PATENT SPECIFICATION

(11) **EP 1 112 074 B1**
(45) Date of publication and mention of the grant of the patent: **21.04.2004**
(21) Application number: 99946325.0
(22) Date of filing: 13.09.1999
(51) Int. Cl.: A61K 31/54, A61P 1/04

(54) **TAUROLIDINE AND/OR TAURULTAM AGAINST INFECTIOUS ULCER OR GASTRITIS**
TAUROLIDIN UND/ODER TAURULTAM ZUR BEHANDLUNG VON INFEKTIÖSEN ULCERA ODER GASTRITIS
TAUROLIDINE ET/OU TAURULTAM DESTINES A LA LUTTE CONTRE LES ULCERES OU LES GASTRITES D'ORIGINE INFECTIEUSE

(30) Priority: 16.09.1998 US 154451; 20.05.1999 US 316115
(43) Date of publication of application: 04.07.2001
(73) Proprietor: Ed Geistlich Söhne AG Für Chemische Industrie, 6110 Wolhusen (CH)
(72) Inventor: PFIRRMANN, Rolf, CH-6000 Lucerne 15 (CH)
(74) Representative: Pett, Christopher Phineas
(86) International application number: PCT/GB1999/003030
(87) International publication number: WO 2000/015232

(56) References cited:
- WO-A-99/34805
- US-A- 5 210 083
- US-A- 5 593 665
- BLENKHARN J.I.: "The antibacterial and anti-endotoxin activity of taurolidine in combination with antibiotics." SURGICAL RESEARCH COMMUNICATIONS, (1987) 2/2 (149-155). , XP002128235

## Description

The present invention relates to the field of treating patients having gastrointestinal ulcers or gastritis caused by microbial infection with certain *Helicobacter sp.* organisms.

Stomach infection with the germ *Helicobacter pylori (H*.*p*.*)* is one of the most frequent infectious diseases in the world; in developing countries, more than 80% of the population is already infected with *H*.*p*. during childhood.

In the past, chronic gastritis with prolonged dyspeptic symptoms in the upper stomach, peptic ulcer, duodenal ulcer (UD) and ventral ulcer (UV) with pain in the upper stomach after meals, and epigastric pain on an empty stomach had been syndromes with unclear etiology. Their pathogenesis had not been clarified in detail. Generally, it can still be said today that there is no peptic ulcer without proteolytic gastric acid. Differential-diagnostic measures usually succeed in differentiating the peptic formation of ulcers from psychogenic gastrointestinal malfunctions, but the final diagnosis depends on X-ray results.

Initially, treatment of chronic gastritis was based on administering antacids, such as magnesium or aluminum compounds, calcium carbonates, alkaline bismuth salts, e.g. bismuth aluminate, or colloidal bismuth salts. However, a high relapse rate of over 80% and side effects, such as the rebound effect of acid secretion, deposits of aluminum and bismuth salts in the tissue, bismuth nephropathy and bismuth encephalopathy, forced the medical field to pursue new paths.

Selective proximal vagotomy with surgical exclusion of the appropriate vagus branches (or the acid-producing stomach sections in the case of relapsing stomach duodenal ulcers) was another medical path that usually went nowhere. The relapse rate usually did not change with this serious surgical procedure.

H₂-receptors are involved in acid secretion. The introduction of the H₂-receptor antagonists cimetidine (Tagamet®) in 1977, and then ranitidine (Zantac®, Sostril™) represented a milestone in medicinal ulcer therapy. This led to rapid pain elimination with the healing of UD and UV. Side effects of long-term therapy, such as infectious diarrhea, persistent hypergastrinemia with germ settlements on the antacid stomach lumen and nitrosamine formation, had to be accepted. Despite progress in acute therapy and the short-term prophylaxis of peptic lesions, the course of ulcers and the relapse rate has not been influenced by the H₂-receptor antagonists.

The currently most potent molecule in the stemming of gastric acid is omeprazol (Antra®). It specifically blocks the H⁺/K⁺ adenosinetriphosphatases (ATP) of the mucosa of the stomach and thus hinders acid secretion.
A suspected disadvantage is that, under permanent hypergastrinemia during omeprazol therapy, a hyperplasia of neuroendocrine cells occurs and can lead to carcinoid tumors. The high effectiveness compared to the H₂-receptor antagonists, however, led to shorter treatment times at lower dosage (20 mg/day). The relapse rate remained unchanged.

Marshall et al. succeeded in 1983-1985 in proving the connection between infection and gastritis through the rediscovery and ability to cultivate the germ *Campylobacter pylori* and through an oral infection in a self-test. This way the actual pathogenic factor of the ulcer was recognized. Initially, the germ was gained from biopsies of the antrum and corpus mucous membrane. *In vitro* cultivation did not succeed until later.

According to examinations conducted by C.S. Goodwin in Perth, Australia, the spiral-shaped *Campylobacter pylori* has little in common with other *Campylobacter* types (different fat composition, different enzyme metabolism, different genetic set-up). Therefore, it had to be renamed *Helicobacter pylori (H.p.).*

*H.p.* is a spiral bent gram-negative germ having rods with lophotric flagellate so-called clusters. Culturing is successful from stomach biopsies (antrum) on accumulation and selective media under microaerobic conditions of 90% N₂, 5% CO₂, 5% O₂ for 3-4 days.

Identification succeeds through additional proof of the enzymes oxidase, catalase, and urease. The germ is able to break down carbamide into ammonia in order to survive in an alkaline environment (cloud) in the acidic environment of the stomach.

H.p. only occurs in humans and is transmitted fecal-orally. The pathogen infects and settles in the mucosa of the stomach.

*Helicobacter heilmanni*, a variation of *H*.*p*., can be found in nearly all cats, dogs, and pigs. It can be passed on to humans. Infected people can develop disorders ranging from ulcers to stomach carcinomas.

Due to the flagellated clusters, *H*.*p*. is particularly mobile and excels through increased adherence to surface cells of the stomach epithelium. The mucous membrane is attacked by proteases of the germ. Vacuolizing cytokinin (VacA), which destroys the epithelium cells, is released.

With an ELISA test, H.p. IgG antibody levels can be proven over an extended period of time, even after infection has subsided. This immune reaction may lead in turn to tissue damage. After the infection, an acute B-gastritis type may develop. When untreated, the gastritis may become chronic, and duodenal and stomach ulcers may occur.

This can then develop into an adenocarcinoma of the stomach. The World Health Organization (WHO) has included *H*.*p*. in Category 4 - Cancerogenes. The germ is to be interpreted as a resistance-weakening factor, which promotes early digestion of the mucosa of the stomach through the hydrochloric-acidic proteolytically active gastric juice.

Modern ulcer therapy has two goals:
1. alleviation of pain; initially, the patient is interested in quick relief of the pain and less in fast healing, i.e. *Helicobacter pylori* eradication; and
2. prevention of complications and relapse rate.

After an *H.p.* infection has healed, reinfection is to be avoided. Flies, for example, can take on *H.p.* bacteria and excrete them in a form that is capable of living in their excreta. Foods must be covered, and general hygienic conditions must be improved as an important preventive measure.

After proof has been secured of *H.p.* following gastroscopy and removal of mucosa of the stomach from the antrum or corpus, eradication of the germ through antibiotics combination therapy is necessary. Serious *H.p.*-related forms of gastritis and underdevelopment should be treated in accordance with cancer prophylaxis - stomach carcinoma and lymphoma (MALT). However, treatment through combination therapy is very difficult. since due to a high relapse rate, mono- and dual-therapy are insufficient. Thus for mono-therapy with bismuth compounds, and for dual-therapy with amoxicillin and omeprazol, the eradication rate is only between 35 and 60%.

Through the combination of bismuth, amoxicillin and metronidazole, healing was achieved for the first time in 85-95% of the cases after a 4-week treatment. The toxicity of this 3-fold combination or 'triple therapy', however, has not yet been clarified, especially when taking the long treatment time of 4 weeks into consideration.

Italian and French triple therapies have also been proposed. The triple therapy is a 7-day treatment with a proton pump inhibitor as well as two antibiotics:
Italian Triple Therapy: clarithromycin and metronidazole;
French Triple Therapy: clarithromycin and amoxicillin.

The medications must be taken twice daily, each in standard dosages. This means that the patient must take 12 tablets a day. Also, there are side effects of the antibiotics, especially diarrhea, taste changes etc. Development of an antibiotic resistance is also potentially disadvantageous (*H.p.* resistance to metronidazole and amoxicillin has already been found).

Accordingly there still remains an urgent need in the art for improved methods of treating gastrointestinal ulcers or gastritis caused by microbial infections in which Helicobacter strains are implicated.

The antimicrobial agents taurolidine and taurultam are described in U.S. Patent No. 5,210,083 in the context of injectable or infusible liquid pharmaceutical compositions additionally containing a parenterally acceptable polyol, optionally together with additives such as buffering electrolytes.

Taurolidine and taurultam have also been proposed for use against some microbial infections of the digestive or intestinal tract, in particular those involving such antibiotic resistant strains as VRE and MRSA - see e.g. Published PCT Application No. WO 99/34805.

The use of these antimicrobial agents in combination with tumour necrosis factor, optionally together with additives such as polyvinylpyrrolidone (PVP), is disclosed in U.S. Patent No. 5,593,665, whilst the antibacterial and anti-endotoxin activities of taurolidine in combination with antibiotics is described by Blenkharn in Surg. Res. Comm. 2, pp. 149-155 (1987).

In one aspect the present invention provides the use of an antimicrobial medicament selected from taurolidine, taurultam or a combination thereof in the manufacture of a therapeutic agent for use in treating infectious gastrointestinal ulcer disease or infectious gastritis disease of microbially infected gastrointestinal tissue in a mammal caused by a microorganism selected from *Helicobacter pylori*, *Helicobacter heilmanni* or a combination thereof, preferably in a human.

Taurolidine and taurultam inactivate cell wall constituents through crosslinking reactions therewith. They are endotoxin non-releasing and/or exotoxin inactivating antimicrobial compounds and are slow-acting bactericides. Taurolidine is a unique antimicrobial agent having an exceptionally broad spectrum of antimicrobial and antibacterial activity including activity against gram positive and gram negative aerobic and anaerobic bacteria. Resistance has not been observed either *in vivo* or *in vitro*.

Taurolidine and taurultam are therefore distinguished from conventional antibiotics as ordinarily understood in the art, i.e. antibiotics that act by attacking, breaking and/or rupturing microbial cell walls (disturbance of murein-biosynthesis, protein-biosynthesis, DNA topology, etc.), resulting in release of microbial toxins from the microbial cells.

Due to its complete bactericidal effectiveness for the destruction of fine cell wall structures, taurolidine is particularly suitable for the eradication of *H.p.* Although not wishing to be bound by theory, adherence of the bacteria to the surface cells of the mucosa of the stomach is believed to be blocked so that the bacteria become apathogenic. Additionally, bacteria cell walls are believed to become net-like so that the release of endotoxins (Zytotoxin VacA) is prevented.

With its microaerobic conditions, taurolidine is especially effective towards anaerobes and *H*.*p*. The minimal inhibitory concentration (MIC) is generally about 70-128 mg/l and the minimal bactericidal concentration (MBC) is generally about 400-600 mg/l. The cell walls of bacteria display no resistance to taurolidine due to its chemical mechanism, so that taurolidine is particularly suitable for the antibacterial mono-therapy of an H.p. infection.

A slight temporary irritation or a burning of the stomach lining has become known as a side effect of taurolidine or taurultam. Through appropriate galenic formulation, such as the utilization of gel-forming agents which settle on the stomach mucosa and create a protective coating, the irritation can be suppressed almost completely. Accordingly medicaments used in accordance with the invention may be manufactured for administration in combination with a pharmaceutically acceptable stomach-coating medication which forms a protective coating on a patient's stomach mucosa so as to prevent stomach irritation by the medicament.

Without being exhaustive, suitable gel-forming agents include: polysaccharides with antiphlogistic properties, protective colloids or polymers such as PVP, methylcellulose, carboxylmethylcellulose, methylhydroxypropylcellulose, gelatin, micro-crystalline collagen fibers, micro-crystalline cellulose, xanthan gum, tragacanth, dextrin, Macrogol 6000, glycogen, micro-crystalline rice starch, absorbent silicate, highly dispersed silicic acid, etc.

Micro-encapsulated compounds which slowly release the medicament in the stomach without irritating the stomach lining can also be produced.

In general, dosages containing 200-1,000 mg taurolidine are possible, with 300-500 mg taurolidine being preferred. This makes it possible to get by with 1-2 tablets, coated tablets, dragees or capsules per day for 5 days to eradicate *H.p.* It is also possible to apply suspensions or syrup with a dosage of about 5 ml twice daily (5 ml syrup typically contains 300 mg taurolidine as the active ingredient).

Another preferred combination is the application of the medicament together with an antacid in order to neutralize gastric acid and thus obtain faster pain alleviation. Accordingly medicaments used in accordance with the invention may be manufactured for administration in combination with a pharmaceutically acceptable gastric acid-neutralizing stomach antacid.

The following antacid compounds (or combinations thereof) can be combined with taurolidine: sodium hydrogen carbonate, hydrotalcite, aluminum-magnesium-hydroxide-carbonate-hydrate, calcium carbonate, magnesium trisilicate, aluminium-magnesium-hydroxide-sulfate-hydrate (Magaldrate), aluminium-magnesium-silicate hydrate, alkaline magnesium carbonate, calcium carbonate, etc.

In general, the antacid is added to the tablet mixture or the granules to fill hard gelatin capsules. It can also be advantageous to add dry extracts of licorice roots with a high content of glyceric acid, chamomile blossom dry extract, peppermint dry extract or other dry plant extracts such as yarrow milfoil, balm, *salvia officinalis* (sage), *foeniculum vulgare* (fennel) that are used for gastritis treatment, to the taurolidine or taurultam compound at a dosage of about 200 mg.

For increased efficacy of taurolidine as well as prolongation of contact time on the mucosa of the stomach, it is advantageous to use taurolidine in micro-crystalline form. Preferred embodiments include formulations with high bioadhesion on mucous cells. The galenic form of preferred embodiments is an oral suspension with the addition of antacids such as Malgaldrate USP XXIII, with added stabilizers, and polymers with bioadhesive properties such as Carbopol™ resins of BF Goodrich.

Substances with high mucous affinity and adhesion are the Carbopol™ types 934P and 971P. In addition to synthetic products, suitable additives include natural polymers and phytogums, such as gum arabic, xanthan gum, pectin, agar, calcium alginate, etc.

In preferred embodiments, disintegration of the tablets in the stomach is swift, preferably into an amorphous to microcrystalline powder.

Suitable tablet disintegrants with high mucous tolerability include natural and semi-synthetic cellulose and starch products, such as microcrystalline cellulose, rice starch, carboxymethyl cellulose and hydroxyethyl cellulose.

The addition of milk powder or blood serum in some cases may be advantageous.

Pharmaceutical compositions comprising an antimicrobial amount of an antimicrobial medicament selected from taurolidine, taurultam or a combination thereof and further comprising a pharmaceutically acceptable stomach-coating medication which forms a protective coating on a patient's stomach mucosa so as to prevent stomach irritation by said antimicrobial medicament, together with a pharmaceutically acceptable gastric acid-neutralizing stomach antacid, constitute a further feature of the invention.

A further preferred combination is the application of taurolidine, taurultam or a combination thereof together with a proton pump inhibitor (PPI) such as omeprazol, rabeprazol or the like. Accordingly these medicaments used in accordance with the invention may be manufactured for administration in combination with a proton pump inhibitor.

PPI's typically are prodrugs which are metabolized at a certain pH. Omeprazol metabolizes at a pH of about 3.9-4.1, whereas rabeprazol metabolizes at a pH of about 4.9. Since taurolidine is less irritating to the stomach at a pH of about 5, rabeprazol is preferred when combined therewith. In particularly preferred embodiments, the sodium salt of rabeprazol is utilized, with preferred dosages of rabeprazol-Na of 10mg or 20mg in the combination.

Pharmaceutical compositions comprising an antimicrobial amount of an antimicrobial medicament selected from taurolidine, taurultam or a combination thereof together with a pharmaceutically acceptable proton pump inhibitor constitute a further feature of the invention.

The invention is further illustrated by way of the following non-limiting Examples:

### Example 1 - Hard Gelatin Capsules

CAPSUGEL, transparent, size 0 elongated

| | | |
|---|---|---|
| Contents | 300 mg | Taurolidine (micro-crystalline) or Taurultam |
| | 6 mg | Talc, Aerosil 200*, Mg-stearates 8:1:1 (additive) |
| | 100 mg | Micro-crystalline rice starch |

### CAPSUGEL, transparent, size 0

| | | |
|---|---|---|
| Contents | 300 mg | Taurolidine or Taurultam |
| | 6 mg | Talc, Aerosil 200*, Mg-stearates 8:1:1 (additive) |
| | 10 mg | Omeprazol, micro-encapsulated, gastric juice-neutral |

| | | |
|---|---|---|
| *Aerosil 200 = Colloidal Silicone | | |

### Example 2 - Tablets or Coated Tablets (direct tablet dispensing)

| | | |
|---|---|---|
| Contents | 300 mg | Taurolidine or Taurultam |
| | 200 mg | Emdex™* |
| | 10 mg | Magnesium stearates |
| | 167 mg | Ranitidine-HCl |
| | 5-10 mg | Disintegrant for tablets** |

| | | |
|---|---|---|
| Contents | 300 mg | Taurolidine or Taurultam |
| | 500 mg | Aluminium-magnesium-silicate-hydrate Aromatic substances and Saccharose |
| | 5-10 mg | Disintegrant for tablets** |

| | | |
|---|---|---|
| Contents | 250 mg | Taurolidine or Taurultam |
| | 500 mg | Calcium Carbonate |
| | 10 mg | Magnesium stearates |
| | 60 mg | Rice or corn starch |
| | 5-10 mg | Disintegrant for tablets** |

| | | |
|---|---|---|
| Contents | 200 mg | Taurolidine |
| | 200 mg | Emdex™ |
| | 10 mg | Magnesium stearates |
| | 10 mg | Crospovidone USP 23/NF 18 |
| | 200 mg | fine dry powder of plants (33% yarrow milfoil herbal, 33% Camomilla flower, 33% Melissa foliate) |

### Example 3 - Taurolidine Tablets (Granule Production with subsequent dispensing)

| | | |
|---|---|---|
| Contents | 350 mg | Taurolidine |
| | 150 mg | Aluminiumhydroxide-magnesium carbonate complex |
| | 80 mg | Corn starch |
| | 45 mg | Kollidon 25 |
| | 5 mg | Aerosil 200*** |
| | 40 mg | Potato starch |
| | 15 mg | Talcum siliconesatum 10% |
| | 15 mg | Slip additives (Talc, Aerosil 200***, Mg-stearates 8:1:1 (additive) Aroma: strawberry, cocoa, saccharine |
| | 370 mg | Alcohol 96% |

| | | |
|---|---|---|
| * Dextrates (Mendell Carmel, New York, U.S.A.) ** For example: Sodium carboxymethylstarch, Carboxymethylcellulose or Crospovidone *** Aerosil 200 = Colloidal Silicone Dioxide | | |

### Production of the Granulates

1. Corn starch and Taurolidine are pre-mixed together with saccharine.
2. Kollidon 25 is dissolved in alcohol.
3. Aluminiumhydroxide-magnesiumcarbonate complex, preblended (see under 1.) as well as the remaining components, such as Aerosil 200, are mixed together in a blunger. The alcohol-damp mass is dried in a vacuum dryer at 30°C, and finally pressed through a 4 mm strainer, then a 2 mm strainer and thus refined into granules.

The dry granules obtained this way are finally mixed well with the potato starch, the slip additive, the talcum siliconesatum 10% and pressed into 700 mg tablets with a 13 mm stamp.

### Example 4 - Syrup

Contents: 100 ml syrup contains:

| | |
|---|---|
| 6,000 mg | Taurolidine micr. or Taurultam |
| 680 mg | Xanthan gum |
| 680 mg | Carboxylmethylcellulose |
| 20,000 mg | Saccharine Ph. Eur. 3 crystalline Vanilla, Aroma, Distilled Water 100ml |

Per dose, 5 ml syrup (300 mg Taurolidine/Taurultam) is taken twice daily.

### Example 5 - Taurolidine Tablets 200 mg

Aluminium Hydroxide Magnesium Carbonate FMA-11 H.D.

| 1. Composition: | |
|---|---|
| **Substance** | **mg/Tablet** |
| Taurolidine | 200 |
| Emdex™ | 90 |
| Starch 1500 | 90 |
| Aluminium Hydroxide Magnesium Carbonate FMA-11 H.D. | 50 |
| Talc | 16 |
| Mg-Stearate | 3 |
| Aerosil 200 | 1 |
| Tablet weight | 450 |

### Example 6 - Taurolidine Tablets 300 mg

Aluminium Hydroxide Magnesium Carbonate FMA-11 H.D.

| 1. Composition: | |
|---|---|
| **Substance** | **mg/Tablet** |
| Taurolidine | 300 |
| Emdex™ | 135 |
| Starch 1500 | 135 |
| Aluminium Hydroxide Magnesium Carbonate FMA-11 H.D. | 75 |
| Talc | 24 |
| Mg-Stearate | 4.5 |
| Aerosil 200 | 1.5 |
| Tablet weight | 675 |

## Claims

1. Use of an antimicrobial medicament selected from taurolidine, taurultam or a combination thereof in the manufacture of a therapeutic agent for use in treating infectious gastrointestinal ulcer disease or infectious gastritis disease of microbially infected gastrointestinal tissue in a mammal caused by a microorganism selected from *Helicobacter pylori, Helicobacter heilmanni* or a combination thereof.

2. Use as claimed in claim 1 wherein said antimicrobial medicament is manufactured for administration in the form of a tablet, capsule, liquid, suspension or syrup.

3. Use as claimed in claim 1 or claim 2 wherein said antimicrobial medicament is manufactured for administration at a dosage equivalent to 200-1,000 mg taurolidine.

4. Use as claimed in claim 3 wherein said antimicrobial compound is manufactured for administration at a dosage equivalent to 300-500 mg taurolidine.

5. Use as claimed in any preceding claim wherein said antimicrobial medicament is manufactured for administration in combination with a pharmaceutically acceptable stomach-coating medication which forms a protective coating on a patient's stomach mucosa so as to prevent stomach irritation by said antimicrobial medicament.

6. Use as claimed in any preceding claim wherein said antimicrobial medicament is manufactured for administration in combination with a pharmaceutically acceptable gastric acid-neutralizing stomach antacid.

7. Use as claimed in any preceding claim wherein said antimicrobial medicament is manufactured for administration in combination with a proton pump inhibitor.

8. Use as claimed in claim 7 wherein said proton pump inhibitor is omeprazol or rabeprazol.

9. Use as claimed in claim 8 wherein said proton pump inhibitor is rabeprazol.

10. A pharmaceutical composition comprising an antimicrobial amount of an antimicrobial medicament selected from taurolidine, taurultam or a combination thereof, and further comprising a pharmaceutically acceptable stomach-coating medication which forms a protective coating on a patient's stomach mucosa so as to prevent stomach irritation by said antimicrobial. medicament, together with a gastric acid-neutralizing stomach antacid.

11. A pharmaceutical composition comprising an antimicrobial amount of an antimicrobial medicament selected from taurolidine, taurultam or a combination thereof together with a pharmaceutically acceptable proton pump inhibitor.

12. A pharmaceutical composition as claimed in claim 11 wherein said proton pump inhibitor.is omeprazol or rabeprazol.

13. A pharmaceutical composition as claimed in claim 12 wherein said proton pump inhibitor is rabeprazol.

## Patentansprüche

1. Verwendung eines antimikrobiellen Medikaments, ausgewählt aus Taurolidin, Taurultam oder einer Kombination hiervon, bei der Herstellung eines therapeutischen Mittels zur Verwendung bei der Behandlung von Magen-Darm-Ulcera oder infektiöser Gastritis von mikrobiell infiziertem Gastrointestinalgewebe in Mammalia, hervorgerufen durch einen Mikroorganismus ausgewählt unter Helicobacter Pylori, Helicobacter Heilmanni oder einer Kombination hiervon.

2. Verwendung nach Anspruch 1, bei der das antimikrobielle Medikament hergestellt wird für die Verabreichung in der Form einer Tablette, Kapsel, Flüssigkeit, einer Suspension oder eines Sirups.

3. Verwendung nach Anspruch 1 oder 2, bei der das antimikrobielle Medikament hergestellt wird für die Verabreichung in einer Dosierung äquivalent zu 200 bis 1000 mg Taurolidin.

4. Verwendung nach Anspruch 3, bei der die genannte antimikrobielle Verbindung hergestellt wird für die Verabreichung in einer Dosierung äquivalent zu 300 - 500 mg Taurolidin.

5. Verwendung nach einem der vorstehenden Ansprüche, bei der das antimikrobielle Medikament hergestellt wird für die Verabreichung in Kombination mit einer pharmazeutisch akzeptablen magenauskleidenden Medikation, die eine Schutzbeschichtung auf Magenschleimhäuten eines Patienten bildet, um eine Magenreizung durch das antimikrobielle Medikament zu verhindern.

6. Verwendung nach einem der vorstehenden Ansprüche, bei der das antimikrobielle Medikament hergestellt wird für die Verabreichung in Kombination mit einem pharmazeutisch akzeptablen magensäureneutralisierenden Magen-Neutralisationsmittel.

7. Verwendung nach einem der vorstehenden Ansprüche, bei der das antimikrobielle Medikament hergestellt wird für die Verabreichung in Kombination mit einem Protonenpumpen-Inhibitor.

8. Verwendung nach Anspruch 7, bei der der Protonenpumpen-Inhibitor Omeprazol oder Rabeprazol ist.

9. Verwendung nach Anspruch 8, bei der der Protonenpumpen-Inhibitor Rabeprazol ist.

10. Pharmazeutische Zusammensetzung mit einer antimikrobiellen Menge eines antimikrobiellen Medikaments, das ausgewählt ist aus Taurolidin, Taurultam oder einer Kombination hiervon, weiterhin enthaltend eine pharmazeutisch akzeptable magenauskleidenden Medikation, die einen schützende Beschichtung auf Magenschleimhäuten eines Patienten bildet, um Magenirritation durch das antimikrobielle Medikament zu verhindern, zusammen mit einem magensäureneutralisierenden Magen-Neutralisationsmittel.

11. Pharmazeutische Zusammensetzung mit einer antimikrobiellen Menge eines antimikrobiellen Medikaments, das ausgewählt ist aus Taurolidin, Taurultam oder einer Kombination hiervon, zusammen mit einem pharmazeutisch akzeptablen Protonenpumpen-Inhibitor.

12. Pharmazeutische Zusammensetzung nach Anspruch 11, bei der der Protonenpumpen-Inhibitor Omeprazol oder Rabeprazol ist.

13. Pharmazeutische Zusammensetzung nach Anspruch 12, bei der der Protonenpumpen-Inhibitor Rabeprazol ist.

## Revendications

1. Application d'un médicament antimicrobien choisi parmi la taurolidine, le taurultame et une de leurs combinaisons à la fabrication d'un agent thérapeutique destiné au traitement des maladies infectieuses à ulcère gastro-intestinal ou des maladies infectieuses de gastrite du tissu gastro-intestinal à infection microbienne d'un mammifère provoquée par un microorganisme choisi parmi *Helicobacter pylori, Helicobacter heilmanai* et une de leurs combinaisons.

2. Application selon la revendication 1, dans laquelle le médicament antimicrobien est fabriqué pour être administré sous forme d'une tablette, d'une capsule, d'un liquide, d'une suspension ou d'un sirop.

3. Application selon la revendication 1 ou 2, dans laquelle le médicament antimicrobien est fabriqué pour être administré avec une dose équivalant à 200 à 1 000 mg de taurolidine.

4. Application selon la revendication 3, dans laquelle le composé antimicrobien est fabriqué pour être administré à une dose équivalant à 300 à 500 mg de taurolidine.

5. Application selon l'une quelconque des revendications précédentes, dans laquelle le médicament antimicrobien est fabriqué pour être administré en combinaison avec un agent médicamenteux de revêtement de l'estomac acceptable pharmaceutiquement qui forme un revêtement protecteur sur la muqueuse de l'estomac du patient pour empêcher l'irritation de l'estomac par le médicament antimicrobien.

6. Application selon l'une quelconque des revendications précédentes, dans laquelle le médicament antimicrobien est fabriqué pour être administré en combinaison avec un agent antiacide pour l'estomac assurant la neutralisation de l'acide gastrique et acceptable pharmaceutiquement.

7. Application selon l'une quelconque des revendications précédentes, dans laquelle le médicament antimicrobien est fabriqué pour être administré en combinaison avec un inhibiteur de pompe à protons.

8. Application selon la revendication 7, dans lequel l'inhibiteur de pompe à protons est l'oméprazol ou le rabéprazol.

9. Application selon la revendication 8, dans laquelle l'inhibiteur de pompe à protons est le rabéprazol.

10. Composition pharmaceutique contenant une quantité antimicrobienne d'un médicament antimicrobien choisi parmi la taurolidine, le taurultame et une de leurs combinaisons, et comprenant en outre un agent médicamenteux de revêtement de l'estomac acceptable pharmaceutiquement qui forme un revêtement protecteur sur la muqueuse de l'estomac du patient pour empêcher l'irritation de l'estomac par le médicament antimicrobien, avec un agent antiacide pour l'estomac assurant la neutralisation de l'acide gastrique.

11. Composition pharmaceutique comprenant une quantité antimicrobienne d'un médicament antimicrobien choisi parmi la taurolidine, le taurultame et une de leurs combinaisons avec un inhibiteur de pompe à protons acceptable pharmaceutiquement.

12. Composition pharmaceutique selon la revendication 11, dans laquelle l'inhibiteur de pompe à protons est l'oméprazol ou le rabéprazol.

13. Composition pharmaceutique selon la revendication 12, dans laquelle l'inhibiteur de pompe à protons est le rabéprazol.
